# EUROPÄISCHE PATENTANMELDUNG

(11) **EP 2 133 111 A1**
(43) Veröffentlichungstag der Anmeldung: **16.12.2009**
(21) Anmeldenummer: 09002966.1
(22) Anmeldetag: 03.03.2009
(51) Int. Cl.: A61M 16/06, G02C 11/00

(54) **Atemgerät mit einem rohrförmigen Brillengestell**

(30) Priorität: 11.06.2008 IT CL20080011
(71) Anmelder: Monsone, Giuseppe, 95131 Catania (IT)
(72) Erfinder: Monsone, Giuseppe, 95131 Catania (IT)

(57) **Zusammenfassung**

Rohrartiges Brillengestell (1) mit Einlass (5) von Gas-Luft.
Am Anfang des Buegels (2) hinter dem Ohr und Austritt (7) in der naehe des Nasenbeins, von hier, mit verschiedenen Anschluessen und Zubehoerteile (8,9), kommt man an die Nasenloecher oder den Mund.

## Beschreibung

Das Geraet besteht aus einem vollkommen rohrartiges u. in seinen ganzen Einzelteilen zerlegbares Brillengestell.

Rohrartiger Buegel mit Gaseinlass am Ende hinter dem Ohr, um dies anschliessend unter der Glaeser, in der Nache des Nasenbeins zu gelangen u. von hier aus,mit einem hautfarbenem Plastikroehrchen von ca. 8 mm an das eine,oder beiden Nasenloecher zu fuehren. Das erlaubt uns die Einatmung durch die Nase (und Ausatmung durch den Mund)von auf Entfernung gefilterte Luft,oder auch Finlass von anreizzenden Gase ,Betaeubungsmitteln, in Extremis auch toedliche Gase,ohne den koerperlichen Zustand zu beschaedigen, fuer Bergsteigen ueber 6000 mt UMS. mit Sauerstoff u. polarisierten Glaeser.

Zwei weiche Ringe an die Nasenloecher koennen diese abdichten um Verschwendung von Gas u. Eintritt von Fremdkoerpern zu verhindern. Ein Miniatemgeraet wird eine gezwungene Einatmung mit grossem Sauerstoff-Ersparnis erlauben. Es werden die antiestaetischen Gesichtsmask en vermieden.

Beim Einatmen auf Null-Quote schicken wir den Lungen Luft mit ca. 21% Sauerstoff, wovon wir ca. 16% wieder ausatmen, zusueglich vieler mehr od. weniger verschmutzter Stoffe, die mit einer normal. Maske wieder teilweise eingefuehrt werden.

Die Herstellung sieht vor die einzelnen Standardteile zu produzieren,die dann, je nach Umstand der einzelnen Person, aufs Mass geschnitten u. angepasst werden.

Fuer das Brillengestell wird ein braun bevorzugt,waehrend Roehrchen u. Nasenanschlusstuecke in durchsichtigem Plastik oder der Hautfarbe des Benuetzers. Wennman aus irgendeinem Grund der Atem-Kreislauf Nase-Mund aendern will,wird der Einlass-Anschluss an einem Mundstueck angebracht und zwischen den Zaehnen gehalten (aehnlich wie beim tauchen);
Wenn man in der Atmungsfase den Mund nicht mitverwickeln will u. wenn beide Nasenloecher frei sind von Materie, Poluepen oder anderes,steht zur Verfuehgung ein anderes Anschlusstueck bestehend aus 2. Einwegmembrane,die das atmen in einem Nasenloch ermoeglichen u. das ausatmen in dem anderen. Durch das Zusammenspiel dreier Einwegmembrane (Ventile) kannjede Kombination zwischen Nasenloch nr.1 u. nr 2 und

Mundstueck, je nach Bedarf, ermoeglicht werden.

Die Zufuhr aufbereiteter Luft kann auch durch einen Druckminderer und mit nachfolgendem,regelbarem Vakuumventil bewerkstelligt werden.
1. Brillengestell,mit Vorderseite aus 2 zusammenzusetzenden Teilen bestehend.
2. 2 Buegel die aufs Mass zu schneiden sind
3. Auskehlung fuer Glaeseranbringung
4. Trennung der Frontseite,aufs Mass zu schneiden u. verbinden
5. Verschiedene Buechsen
6. 2 Anschluesse fuer Gaseintritt,auch einzeln zu benuetzen
7. Verlaengerung vom Gestell zur Nasenloecher
8. Mundanschlusstueck in kleinem Mass u. Einwegventil
9. Nasenanschlusstuecke
10. Verschiedene Stopfen
11. Bandverschluss mit moeglicher Abkuerzung des Buegels
12. Dichtungsringe fuer die Nase,mit conischem oder rundem Querschnitt
13. Membranartige und umkehr bares Einwegventil

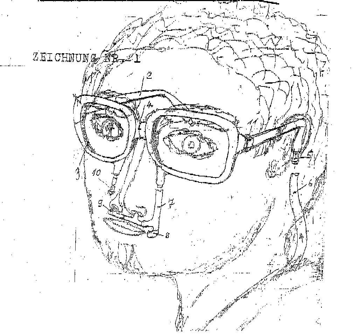

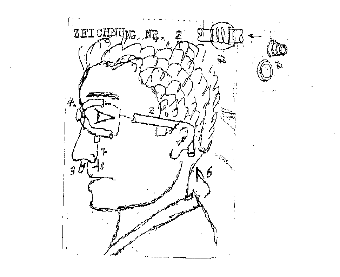

## Patentansprüche

1. EINFUHR IN DIE NASE UND-ODER, DEN MUND IRGENDEINER GASFOERMIGEN SUBSTANZ,DURCH EIN BRILLENGESTELL. DISER EINFUHR KANN GEPRAESST ODER NICHT, GEFILTERT ODER NICHT, MIT ODER OHNE EINWEGVENTILE ERFOLGEN.
